# FASCICULE DE BREVET EUROPEEN

(11) **EP 1 480 610 B1**
(45) Date de publication et mention de la délivrance du brevet: **09.01.2008**
(21) Numéro de dépôt: 03715073.7
(22) Date de dépôt: 05.02.2003
(51) Int. Cl.: A61K 8/99, A61K 35/56, A61K 35/66, A61Q 17/00, A61Q 19/00

(54) **UTILISATION D UN COMPOSE INDUCTEUR DE HSP AFIN DE LIMITER LES EFFETS SECONDAIRES DES RETINOIDES**
VERWENDUNG EINER HSP INDUZIERENDEN VERBINDUNG ZUR BEGRENZUNG SEKUNDÄRER WIRKUNGEN VON RETINOIDEN
USE OF A HSP INDUCING COMPOUND TO LIMIT SECONDARY EFFECTS OF RETINOIDS

(30) Priorité: 08.02.2002 FR 0201539; 13.02.2002 FR 0201746
(43) Date de publication de la demande: 01.12.2004
(73) Titulaire: Société d'Extraction des Principes Actifs ( Vincience SA), 06410 Biot (FR)
(72) Inventeur: DAL FARRA, Claude, F-06650 Opio (FR); DOMLOGE, Nouha, F-06560 Valbonne (FR); PEYRONEL, Dominique, F-13014 Marseille (FR)
(74) Mandataire: Macquet, Christophe
(86) Numéro de dépôt international: PCT/FR2003/000359
(87) Numéro de publication internationale: WO 2003/066014

(56) Documents cités:
- WO-A-93/06818
- WO-A-99/38483
- FR-A- 2 809 308
- FR-A- 2 810 241
- CUCUMEL K ET AL: "Artemia extract induces Hsp70 in human cells and enhances cell protection from stress." JOURNAL OF INVESTIGATIVE DERMATOLOGY, vol. 117, no. 2, août 2001 (2001-08), page 454 XP002218532 62nd Annual Meeting of the Society for Investigative Dermatology;Washington, DC, USA; May 09-12, 2001 ISSN: 0022-202X
- TOZI,P. ET AL.: "reduction of heat schock protein 70 after prolonged treatment with retinoids: biological and clinical implications" AMERICAN JOURNAL OF HEMATOLOGY, vol. 56, no. 3, novembre 1997 (1997-11), pages 143-150, XP009074662

## Description

La présente invention se rapporte à l'utilisation d'une quantité efficace d'au moins un composé inducteur de protéines HSP dans ou pour la préparation d'une composition, le composé ou la composition étant destinés à limiter les effets secondaires des rétinoïdes.

La présente invention se rapporte aussi à une composition cosmétique ou pharmaceutique ou dermatologique, comprenant au moins un rétinoïde et au moins un inducteur de protéines HSP, ainsi qu'à son utilisation.

La famille des rétinoïdes regroupe des composés présentant un profil d'activité biologique analogue à celui de l'acide rétinoïque *tout-trans* (encore appelé vitamine A acide, trétinoïne ou acide *all-trans* rétinoïque) ou de l'acide 9-cis-rétinoïque (encore appelé isotrétinoïdes). Ils modulent l'expression de certains gènes par le biais des récepteurs de l'acide rétinoïque (récepteurs RAR).

Les compositions cosmétiques et/ou dermatologiques à base de ces rétinoïdes ont connu un important développement au cours de ces dernières années. Leurs activités sont décrites, entre autres, au niveau de la différenciation et de la prolifération cellulaire. Parmi les composés représentatifs de ce groupe, on peut citer, par exemple, l'acide rétinoïque ou le rétinal, sous forme de leurs différents isomères.

Leurs utilisations pharmaceutiques sont très nombreuses, notamment comme anti-cancéreux. Leur usage est courant aussi dans les affections rhumatismales, cardiovasculaires, respiratoires ou encore dermatologiques.

Ainsi, par exemple, au niveau dermatologique et cosmétique, l'acide rétinoïque est utilisé pour le traitement de l'acné ainsi que le décrit le brevet US 5,034,228. Par ailleurs, dans le brevet US 5,719,195, l'acide rétinoïque 11-cis est utilisé pour le traitement du psoriasis.

De plus, il a été montré que les rétinoïdes, et en particulier l'acide rétinoïque, ont un effet favorable sur l'amélioration de l'apparence et de l'état de la peau, et permettent de lutter contre les signes du vieillissement. Une méthode de lutte contre le photo-vieillissement a, par exemple, été décrite dans le brevet EP 0 230498, ainsi que dans le brevet EP 027404 où l'acide rétinoïque 13-cis est utilisé pour le traitement des peaux âgées.

Cependant, il est clairement établi que l'utilisation de rétinoïdes présente de nombreux effets secondaires très gênants. En particulier, leur usage est extrêmement irritant pour la peau et provoque, entre autres, des phénomènes inflammatoires (WO 93/06818) et font chuter les taux de HSP70 (Tosi et al., Am.J. Hemat , 1997, vol 56 no.3, pp 143-150).

Bien que plusieurs méthodes et formulations aient été proposées pour réduire les effets secondaires produits par l'utilisation de ces rétinoïdes, aucune solution n'est actuellement totalement satisfaisante. On constate donc que subsiste le besoin d'un produit nouveau, exerçant une action sur les traitements avec des rétinoïdes afin de compenser leurs effets secondaires.

Or, la demanderesse a trouvé, de façon surprenante et inattendue, qu'une quantité efficace d'un composé inducteur de *Heat Shock Protreins* (HSP) exerce une action bénéfique sur les peaux qui ont été traitées avec des rétinoïdes, notamment ce composé inducteur de HSP permet d'éviter les effets secondaires gênants que provoque l'utilisation de rétinoïdes.

Les HSP sont parmi les protéines les plus anciennes et les mieux conservées. Il en existe des homologues dans toutes les espèces et dans tous les règnes vivants. Par exemple, dnaK, un seul gène chez la bactérie, est devenu le complexe multigénique de la famille des HSP70 chez *Saccharomyces sp.,* chez la drosophile et chez l'homme.

Si les HSP sont apparues très tôt au cours de l'évolution, c'est en raison des conditions environnementales extrêmes auxquelles étaient confrontés les organismes. Au cours de l'évolution, les protéines HSP ont conservé leur rôle vital de protection contre le stress, elles ont également pris en charge de nouvelles fonctions comme, par exemple, le transport des protéines à l'intérieur de la cellule.

Le mécanisme essentiel de défense de tout organisme est la conservation de son matériel génétique, protéique et membranaire. Pour cela, les protéines HSP, dites protéines de stress ou *Heat Shock Proteins* (HSP), ont pour rôle de protéger ou de réparer le matériel génétique, les peptides et les protéines détériorés lors d'un stress. La synthèse des HSP est donc un moyen de défense généralisé, développé par la cellule pour lutter contre la grande diversité des agressions possibles.

Les HSP sont aussi appelées molécules chaperonnes car elles s'associent à d'autres molécules, soit dénaturées, soit en cours de biosynthèse. Ces interactions moléculaires ont pour but de prévenir l'agrégation de protéines altérées ou en cours de biosynthèse, de participer à l'acquisition de leur structure tridimensionnelle, d'éliminer les protéines anormales et de participer au transport des protéines depuis le cytoplasme vers la membrane plasmique ou vers des organites tels que les mitochondries, le réticulum endoplasmique, les lysosomes ou le noyau. En interagissant avec certaines protéines, les HSP vont minimiser la probabilité pour ces protéines d'interagir avec d'autres de façon inappropriée. Par ailleurs, les HSP reconnaissent et se lient aux molécules qui ne sont pas dans une conformation native, soit après un stress dénaturant, soit sur les protéines en cours de synthèse, de repliement, d'assemblage, soit encore car elles ne sont pas localisées dans le bon compartiment subcellulaire. Les protéines HSP jouent donc un rôle primordial dans la protection contre tous les phénomènes de stress cellulaire.

Au niveau de la peau, des études ont montré la présence de HSP27, HSP47, HSP60, HSP70 et HSP90. Les HSP27 et HSP90 sont exprimées au niveau épidermique dans les couches basale et suprabasales, les HSP60 au niveau suprabasal et les HSP70 sont exprimées au niveau épidermique (Boechncke et coll., 1994). D'autres études ont montré la présence de HSP47 au niveau épidermique. FR 2810241 propose des inducteurs de HSP, par exemple de HSP70, sous la forme par exemple d'extraits d'Artemia salina,

A la connaissance de la demanderesse, il n'a jamais été décrit dans l'art antérieur l'utilisation de composé inducteur de HSP afin de limiter les effets secondaires des rétinoïdes.

L'invention a donc pour objet l'utilisation d'une quantité efficace d'au moins un composé inducteur de *Heat Shock Proteins* (HSP) dans ou pour la préparation d'une composition, le composé ou la composition étant destinés à limiter les effets secondaires des rétinoïdes.

Par effets secondaires des rétinoïdes, on entend tous les effets secondaires gênants qui se produisent à cause de l'administration d'un composé de la famille des rétinoïdes comme, par exemple, les phénomènes inflammatoires ou irritants. Plus généralement le terme "effet secondaire gênant" inclus toutes les manifestations qui incommodent plus ou moins l'individu qui fait usage de rétinoïdes. On peut donc encore citer, à titre d'exemple, les rougeurs de la peau ou les démangeaisons.

Par la suite, le terme "inducteur de *Heat Shock Proteins* (HSP)" doit s'entendre comme étant un composé qui, lorsqu'il est appliqué sur la peau ou les cellules, va induire une synthèse des protéines HSP *(Heat Shock Proteins)* ou va permettre de maintenir un taux élevé de HSP. La plupart du temps, l'inducteur de protéines HSP selon l'invention va permettre d'augmenter la teneur en HSP au niveau cellulaire.

Le composé inducteur de protéines HSP va induire des protéines qui appartiennent à une ou à plusieurs des familles de HSP parmi lesquelles on peut citer les petites HSP (10-30 kDa), les HSP40, les HSP60, les HSP70, les HSP90 ou encore les HSP100-110.

Les rétinoïdes selon l'invention sont choisis dans le groupe comprenant l'acide tout-trans rétinoïque, l'acide 13-cis-rétinoïque, l'acide 9-cis-rétinoïque, les rétinaldéhydes ou encore leurs sels ou leurs esters.

Les sels des rétinoïdes selon l'invention sont choisis dans le groupe comprenant les sels de sodium, de potassium, d'ammonium ou d'alcanolamine en C2-C30.

Les rétinoïdes de l'invention ne comprennent pas les composés tels que la vitamine A (ou rétinol), qui est un composé couramment utilisé dans les compositions cosmétiques mais dont les applications différent.

Le composé inducteur de protéines HSP est un extrait du zooplancton obtenu à partir de l'espèce *Artemia salina.* Selon un mode de réalisation préféré de l'invention, le composé inducteur de protéines HSP est obtenu à partir de l'espèce *Artemia salina* en état de dormance. Selon un autre mode de réalisation préféré de l'invention, le composé inducteur de protéines HSP est un extrait *d'Artemia salina* contenant au moins 150 mg/litre de diguanosine tétraphosphate.

Un autre objet de l'invention est l'utilisation d'une quantité efficace d'au moins un composé inducteur de HSP dans ou pour la préparation d'une composition, le composé ou la composition étant destinés à maintenir un taux physiologique de protéines HSP dans les cellules et/ou dans la peau, lors d'un traitement par des rétinoïdes.

Plus particulièrement selon l'invention, le composé ou la composition permettent de maintenir un taux physiologique de protéines HSP dans les cellules âgées et/ou dans la peau âgée, lorsque celles-ci sont traitées par des rétinoïdes.

On entend par un "taux physiologique de *Heat Shock Proteins"* un taux d'expression des protéines HSP qui n'excède pas la normale, c'est-à-dire des concentrations que l'on trouve naturellement dans les cellules et dont les proportions n'induisent pas de cytotoxicité.

L'implication des protéines HSP dans la protection cellulaire et cutanée est très importante ; en effet les protéines HSP jouent un rôle primordial dans la protection des cellules contre le stress. En rétablissant ainsi un taux physiologique de protéines HSP, notamment lorsque les cellules sont traitées avec des rétinoïdes, les composés selon l'invention vont pouvoir faire bénéficier des avantages des rétinoïdes sans toutefois entraîner de diminution des défenses de la peau, c'est-à-dire sans avoir les effets néfastes des rétinoïdes.

Un autre objet de l'invention est l'utilisation d'une quantité efficace d'au moins un composé inducteur de HSP dans ou pour la préparation d'une composition, le composé ou la composition étant destinés à régulariser le taux de cytokines inflammatoires lors d'un traitement des cellules et/ou de la peau par des rétinoïdes.

Par les cytokines inflammatoires on entend la classe de molécules qui vont jouer un rôle important dans le déclenchement des phénomènes de l'inflammation. Elles comprennent, notamment, les interleukines, le TNF, les interférons ainsi que de nombreuses autres substances apparentées.

Le composé inducteur de protéines HSP va faire diminuer le taux de cytokines comme, par exemple, le taux d'interleukine-1, de manière à ne pas déclencher de réponse inflammatoire. Le taux d'interleukine sera ramené à son taux intracellulaire basal. Ainsi, le taux de cytokines inflammatoires, nettement surexprimé lors des traitements par l'acide rétinoïque, sera modulé lors d'une association avec un composé inducteur de protéines HSP. Ce composé permet donc de diminuer l'inflammation induite par les rétinoïdes.

Par ailleurs, un autre objet de l'invention est l'utilisation d'une quantité efficace d'au moins un composé inducteur de HSP dans ou pour la préparation d'une composition, le composé ou la composition étant destinés à protéger les cellules et/ou la peau des stress d'origines extérieures durant les thérapies utilisant des rétinoïdes.

On entend par stress d'origine extérieure tous les types d'agressions que peuvent subir la peau et/ou les cellules cutanées. Ces agressions peuvent être d'origine chimique, physique, biologique ou thermique. A titre d'exemple, on peut citer des agressions telles que la pollution, les UV, les métaux lourds, les frottements, l'eau à forte concentration de calcaire, les variations de température ou encore les produits à caractère irritant tels que les tensioactifs, les conservateurs, les solvants ou les parfums.

Lors des traitements par les rétinoïdes, la peau et/ou les cellules seront beaucoup plus sensibles aux agressions, elles développeront plus facilement des réactions inflammatoires lorsqu'elles seront soumises à des stress d'origines extérieures. L'utilisation d'un composé inducteur de protéines HSP permettra de mieux protéger la peau et/ou les cellules, c'est-à-dire, par exemple, il permettra de diminuer la réponse immunitaire occasionnée par le stress.

Selon un autre aspect, l'invention a pour objet une composition caractérisée en ce qu'elle contient, comme principe actif, dans un milieu cosmétiquement ou pharmaceutiquement acceptable, au moins un rétinoïde et au moins un inducteur de protéines HSP.

La composition selon l'invention peut être une composition cosmétique et/ou dermatologique et/ou pharmaceutique. Préférentiellement selon l'invention, la composition est une composition cosmétique ou dermatologique, car elle est destinée à améliorer l'aspect et les performances cutanées générales de l'individu qui en fait usage.

La composition selon l'invention est préférentiellement une composition cosmétique et/ou dermatologique adaptée à l'administration par voie topique cutanée comprenant un milieu cosmétiquement ou dermatologiquement acceptable.

Il est bien évident que l'invention s'adresse aux mammifères en général et plus particulièrement aux êtres humains.

L'inducteur de protéines HSP de la composition de l'invention est un extrait de zooplanctons de l'espèce *Artemia salina.* Plus particulièrement l'extrait d'Artémia est obtenu à partir de l'espèce *Artemia salina* en état de dormance. On entend par "état de dormance" un état dans lequel l'organisme suspend l'ensemble de ces fonctions métaboliques de façon à s'isoler du milieu extérieur.

Selon un autre mode de réalisation avantageux de la composition de l'invention, l'extrait *d'Artemia salina* contient au moins 150 mg/litre de diguanosine tétraphosphate.

Pour donner un ordre de grandeur, on peut utiliser, dans la composition de l'invention, le composé inducteur de protéines HSP en une quantité représentant de 0,0001 % à 20 % du poids total de la composition, et préférentiellement en une quantité représentant de 0,001 % à 5 % du poids total de la composition.

La concentration en composés rétinoïdes peut être comprise entre 0,0001 % et 5 % du poids total de la composition, et préférentiellement la concentration en composés rétinoïdes est comprise en une quantité représentant de 0,001 % à 1 % du poids total de la composition.

Les rétinoïdes de la composition sont choisis parmi le groupe comprenant l'acide tout-trans rétinoïque, l'acide 13-cis-rétinoïque, l'acide 9-cis-rétinoïque, les rétinaldéhydes, leurs sels et/ou leurs esters.

Selon un mode de réalisation avantageux de l'invention, l'inducteur de protéines HSP précité est préalablement solubilisé dans un ou plusieurs solvants cosmétiquement ou pharmaceutiquement acceptables comme l'eau, l'éthanol, le propanol ou l'isopropanol, le propylène glycol, le butylène glycol, le dipropylène glycol, les diglycols éthoxylés ou propoxylés, les polyols cycliques, la vaseline, une huile végétale ou tout mélange de ces solvants.

Selon encore un autre mode de réalisation avantageux de l'invention, l'inducteur de protéines HSP précité peut être préalablement solubilisé dans un vecteur cosmétique ou pharmaceutique comme les liposomes ou adsorbés sur des polymères organiques poudreux, des supports minéraux comme les talcs et bentonites, et plus généralement solubilisés dans ou fixés sur tout vecteur cosmétiquement ou pharmaceutiquement acceptable.

Quelle que soit la forme de l'invention, la composition selon l'invention peut être ingérée, injectée ou appliquée sur la peau (sur toute zone cutanée du corps), les cheveux, les phanères ou les muqueuses. Selon le mode d'administration, la composition selon l'invention peut se présenter sous toutes les formes galéniques normalement utilisées.

Préférentiellement, les compositions selon la présente invention se présenteront sous une forme galénique adaptée à l'administration par voie topique cutanée. Elles couvrent toutes les formes cosmétiques ou dermatologiques. Ces compositions doivent donc contenir un milieu cosmétiquement ou dermatologiquement acceptable, c'est-à-dire compatible avec la peau, les poils ou les cheveux.

Ces compositions peuvent notamment se présenter sous forme d'une solution aqueuse, hydralcoolique ou huileuse ; d'une émulsion huile-dans-eau, eau-dans-huile ou émulsions multiples ; elles peuvent aussi se présenter sous forme de crèmes, de suspensions ou encore de poudres, adaptées à une application sur la peau, les muqueuses, les lèvres et/ou les cheveux.

Ces compositions peuvent être plus ou moins fluides et avoir l'aspect d'une crème, d'une lotion, d'un lait, d'un sérum, d'une pommade, d'un shampooing, d'un gel, d'une pâte ou d'une mousse. Elles peuvent aussi se présenter sous forme solide, comme un stick ou être appliquées sur la peau sous forme d'aérosol. Elles peuvent être utilisées comme produit de soin et/ou comme produit de maquillage de la peau.

Pour l'injection, la composition selon l'invention peut se présenter sous forme de lotion aqueuse, huileuse ou sous forme de sérum. Pour l'application sur les yeux, la composition peut se présenter sous forme de gouttes et pour l'ingestion, elle peut se présenter sous forme de capsules, de granulés, de sirops ou de comprimés.

Ces compositions comprennent, en outre, tout additif usuellement utilisé dans le domaine d'application envisagé ainsi que les adjuvants nécessaires à leur formulation, tels que des solvants, des épaississants, des diluants, des anti-oxydants, des colorants, des filtres solaires, des agents auto-bronzants, des pigments, des charges, des conservateurs, des parfums, des absorbeurs d'odeur, des actifs cosmétiques ou pharmaceutiques, des huiles essentielles, des vitamines, des acides gras essentiels, des tensioactifs, des polymères filmogènes, etc....

Dans tous les cas, l'homme du métier veillera à ce que ces adjuvants ainsi que leurs proportions soient choisis de telle manière à ne pas nuire aux propriétés avantageuses recherchées de la composition selon l'invention. Ces adjuvants peuvent, par exemple, correspondre de 0,01 à 20 % du poids total de la composition.

Lorsque la composition de l'invention est une émulsion, la phase grasse peut représenter de 5 à 80 % en poids et de préférence de 5 à 50 % en poids par rapport au poids total de la composition. Les émulsionnants et co-émulsionnants utilisés dans la composition seront choisis parmi ceux classiquement utilisés dans le domaine considéré. Par exemple, ils peuvent être utilisés en une proportion allant de 0,3 à 30 % en poids, par rapport au poids total de la composition.

Bien entendu, l'homme du métier veillera à choisir les éventuels composés complémentaires, actifs ou non-actifs, et/ou leurs quantités, de telle sorte que les propriétés avantageuses du mélange ne soient pas ou sensiblement pas, altérées par l'adjonction envisagée.

Les compositions selon l'invention trouvent une application notamment comme composition cosmétique ou pharmaceutique pour la peau, les muqueuses et/ou les semi-muqueuses, mais aussi comme composition cosmétique pour les cheveux et/ou les poils.

Elles trouvent une application toute particulière en tant que produit de protection et/ou de soin de la peau.

On peut également envisager une application dans le domaine des compositions de maquillage de la peau du visage et du corps, telles que les rouges à lèvres, les fond de teint, les crèmes teintées, les sticks anti-cemes, les compositions anti-solaires ou de bronzage artificiel.

Les compositions, objet de l'invention, trouvent leur application dans grand nombre de traitements notamment cosmétiques ou dermatologiques, et elles peuvent constituer une composition cosmétique, notamment pour le traitement, la protection, le soin, le démaquillage et/ou le nettoyage de la peau, des lèvres et/ou des cheveux, et/ou pour le maquillage de la peau, des lèvres, des cils et/ou du corps.

La composition selon l'invention peut aussi être utilisée dans les compositions pharmaceutiques et dermatologiques destinées à tout usage notamment aux applications topiques.

La composition selon l'invention peut également consister en des préparations solides comprenant également des savons ou des pains de nettoyage. La composition peut être aussi conditionnée sous forme d'une composition pour aérosol comprenant également un agent propulseur sous pression. La composition peut être aussi à usage bucco-dentaire, par exemple une pâte de dentifrice.

La composition de l'invention peut aussi être une composition cosmétique ou dermatologique ou pharmaceutique destinée à une administration par voie orale. Pour une administration par voie orale, la composition selon l'invention peut se présenter sous toutes formes adaptées, particulièrement sous forme d'une solution buvable, d'un sirop, d'un comprimé, d'une dragée, d'une gélule ou encore d'un aliment ou complément nutritionnel.

Selon l'invention, on peut, entre autres, ajouter à la composition de l'invention d'autres agents actifs destinés notamment à la prévention et/ou au traitement des manifestations cutanées du vieillissement et/ou à la protection de la peau et/ou des cheveux contre les agressions extérieures.

Un autre aspect de l'invention concerne l'utilisation de la composition, telle que définie précédemment, afin de lutter de manière curative et/ou préventive contre les manifestations cutanées du vieillissement.

Par modifications cutanées du vieillissement on entend toutes modifications de l'aspect extérieur de la peau dues au vieillissement comme, par exemple, les rides et ridules, la peau flétrie, la peau molle, la peau amincie, le manque d'élasticité et/ou de tonus de la peau, la peau terne et sans éclat mais également toutes modifications internes de la peau qui ne se traduisent pas systématiquement par un aspect extérieur modifié comme, par exemple, toutes dégradations internes de la peau consécutives à une exposition aux rayonnements ultra-violets.

La composition selon l'invention peut aussi être utilisée afin de protéger les cellules et/ou la peau contre tous les types d'agressions d'origines extérieures qu'elles peuvent subir.

On entend par le terme "agression extérieure" les agressions que peut produire l'environnement. Ces agressions peuvent être d'origine chimique, physique, biologique ou thermique. A titre d'exemple, on peut citer des agressions telles que la pollution, les UV, les métaux lourds, les frottements, l'eau à forte concentration de calcaire, les variations de température ou encore les produits à caractère irritant tels que les tensioactifs, les conservateurs, les solvants ou les parfums.

La composition selon l'invention peut aussi être utilisée pour tous les types de soins traditionnellement appliqués à la peau. Par les soins, on entend toutes les actions destinées à conserver ou à rétablir un bon fonctionnement de la peau ou encore tout moyen qui sert à préserver ou à améliorer son apparence et/ou son aspect.

Ainsi le soin inclus l'hydratation, l'apaisement, la protection contre tous types d'agression, notamment la protection solaire, la lutte et la prévention des manifestations du vieillissement, notamment les manifestations cutanées du vieillissement.

Les avantages apportés par cette composition permettent d'envisager sereinement l'utilisation des rétinoïdes, sachant que les défenses de la peau ne seront pas affaiblies mais au contraire renforcées.

Selon un autre aspect, la présente invention concerne également un procédé de traitement cosmétique de la peau dans lequel on applique, sur la peau et/ou sur les cheveux, la composition définie précédemment afin de limiter les effets secondaires des rétinoïdes.

La présente invention concerne encore un procédé de traitement cosmétique de la peau destiné à traiter les signes cutanés du vieillissement, consistant à appliquer, sur la peau et/ou les muqueuses, une composition cosmétique telle que définie précédemment.

L'invention concerne un procédé de traitement cosmétique afin de protéger la peau et/ou les cheveux contre tous les types d'agressions extérieures, consistant à appliquer, sur la peau et/ou les cheveux et/ou les muqueuses une composition cosmétique telle que définie précédemment.

L'invention concerne un procédé cosmétique afin d'apporter des soins à la peau et/ou aux cheveux, consistant à appliquer, sur la peau et/ou les cheveux et/ou les muqueuses une composition cosmétique telle que définie précédemment.

Le procédé de traitement cosmétique de l'invention peut être mis en oeuvre notamment en appliquant les compositions cosmétiques telles que définies ci-dessus, selon la technique d'utilisation habituelle de ces compositions, par exemple : application de crèmes, de gels, de sérums, de lotions, de laits, de shampooings ou de compositions anti-solaires, sur la peau ou sur les cheveux, ou encore, application de dentifrice sur les gencives.

La figure 1 illustre le résultat d'un Immunoblotting des protéines HSP sur des fibroblastes humains après 1 jours de culture (ligne 1) et après 24 jours de culture (lignes 2, 3 et 4).

La figure 2 illustre le résultat d'un Immunoblotting des protéines HSP sur des fibroblastes humains après 1 jours de culture (ligne 1) et après 51 jours de cultures (lignes 2, 3, 4 et 5).

La figure 3 représente l'évaluation de la quantité d'IL-1 alpha dans les cellules HaCat et dans les fibroblastes.

La figure 4 représente l'évaluation de la quantité d'IL-1 bêta dans des fibroblastes traités avec de l'ATRA et/ou avec l'extrait de l'exemple 1, après une irradiation avec 100 mJ/cm2 d'UVB.

### Exemple 1 : Procédé d'obtention d'un inducteur de protéines HSP

Dans une première étape, 50 grammes "d'oeufs" de zooplanctons de l'espèce *d'Artemia salina* sont réhydratés pendant des durées de 30 minutes à 6 heures, à une température comprise entre 30°C à 65°C, dans 1 litre d'un milieu adapté, principalement constitué d'eau, et à un pH compris entre 4 et 7.

Ces oeufs sont, par la suite, broyés de façon à obtenir un extrait homogène. Le contenu intra-cytoplasmique obtenu est centrifugé et filtré. L'extrait est enfin stérilisé par filtration stérilisante et chauffage à 65°C.

Un dosage HPLC est réalisé afin de confirmer une teneur en diguanosine tétraphosphate supérieure à 150 mg/litre.

L'extrait est ensuite stabilisé : il est traité et stérilisé pour se conformer aux exigences cosmétiques (couleur, odeur, aspect, stérilité...). Une concentration variable de glycérol est ensuite ajoutée afin de stabiliser les protéines. L'extrait de zooplancton contient alors de 0 à 15 % de glycérol.

L'extrait est ensuite utilisé dans des préparations contenant de 0,1 % à 15 % de l'extrait de zooplancton.

### Exemple 2 - Effets des rétinoïdes et de l'extrait de l'exemple 1 sur l'expression in vitro des protéines HSP.

Des études par immunoblotting ont été conduites sur des fibroblastes humains cultivés respectivement pendant 24 jours et 51 jours, à 37°C et à 5 % de CO₂ dans une étuve humide, dans différentes conditions.

Plusieurs conditions ont été testées :
- Condition contrôle (Cellules non traitées),
- Cellules traitées avec 10⁻⁷ M d'acide *all-trans* rétinoïque (ATRA),
- Cellules traitées avec 3 % de l'extrait de l'exemple 1,
- Cellules traitées avec 10⁻⁷ M d'acide *all-trans* rétinoïque (ATRA) et avec 3 % de l'extrait de l'exemple 1.

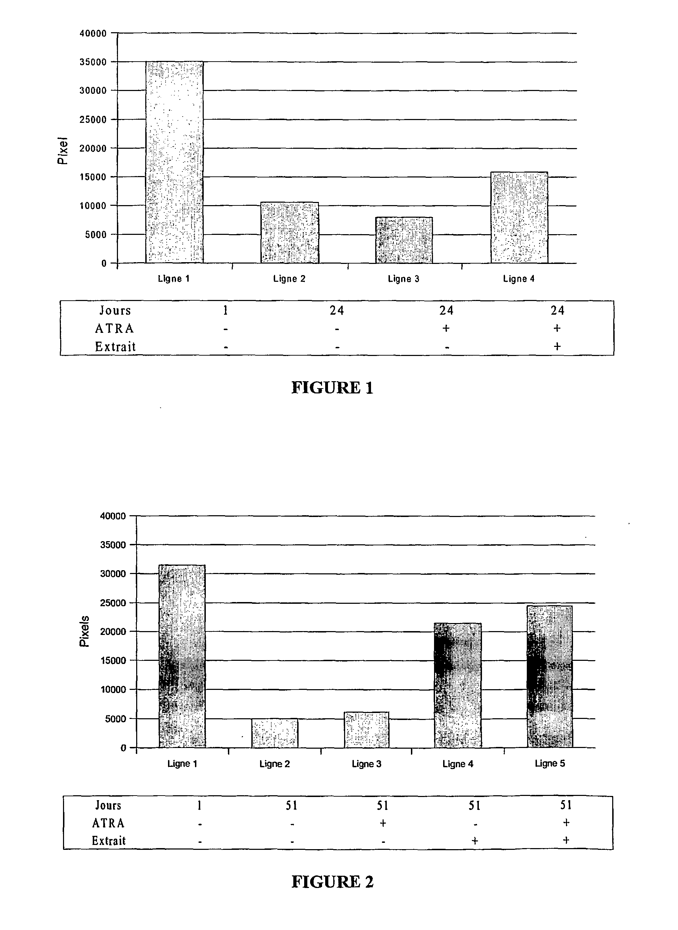

Les résultats obtenus, illustrés par les figures 1 et 2, représentent les quantités de protéines HSP obtenues par immunoblotting, suivant les différentes conditions de culture.

Les résultats présentés à la figure 1 démontrent que, sur les fibroblastes jeunes, c'est-à-dire cultivés pendant 24 jours, l'expression des HSP 70 est diminuée par le traitement avec l'acide rétinoïque. Par contre, l'association avec l'extrait de l'exemple 1 permet de rétablir un taux physiologique de protéines HSP.

D'autre part, les résultats présentés à la figure 2 mettent en évidence que les fibroblastes vieillissants, c'est-à-dire cultivés pendant 51 jours, expriment beaucoup moins de HSP 70 que les fibroblastes plus jeunes. Dans ce cas, le traitement avec l'acide rétinoïque augmente légèrement l'expression des HSP 70, alors que le traitement avec l'extrait d'Artémia de l'exemple 1 en association, ou non, avec l'acide rétinoïque rétablit un taux de HSP proche de celui présent dans les fibroblastes jeunes.

Ces résultats démontrent ainsi que l'association entre l'extrait de l'exemple 1 et des rétinoïdes permet de maintenir un taux physiologique de protéines HSP dans les cellules jeunes et dans les cellules âgées.

### Exemple 3 - Effets des rétinoïdes et de l'extrait de l'exemple 1 sur l'expression cutanée des protéines HSP.

Des études *ex vivo* ont permis, par immunomarquage, de mettre en évidence l'expression des protéines HSP 70 sur des échantillons de peau mis en culture pendant 5 jours. Les échantillons ont été traités ou non avec de l'acide rétinoïque à 10⁻⁷ M et avec l'extrait de l'exemple 1 à 3 %. Les traitements ont été réalisés deux fois par jour pendant les 5 jours de culture. Les échantillons de peau ont été fixés au formol salé à 9 % pendant 10 heures puis inclus dans la paraffine de façon automatisée. Des coupes de 2 à 3 µm ont ensuite été réalisées afin de procéder à l'immunomarquage.

Les résultats obtenus démontrent que les peaux traitées avec l'acide rétinoïque expriment nettement moins de protéines HSP 70 que les peaux non traitées. En revanche, les peaux traitées avec une association d'acide rétinoïque et d'extrait selon l'exemple 1 expriment un taux de protéines HSP 70 comparable à celui observé dans les peaux non traitées.

Ainsi, l'extrait de l'exemple 1 permet, au niveau cutané, de rétablir une teneur en protéines HSP d'ordre physiologique, et ce, malgré la diminution apportée par le traitement par les rétinoïdes.

### Exemple 4 - Effets des rétinoïdes et de l'extrait de l'exemple 1 sur l'expression cutanée des protéines HSP suite à un stress UV.

Les expériences réalisées dans l'exemple 3 ont été reconduites, mais les peaux ont été irradiées avec des doses de UVB de 200 mJ/cm².

Les résultats obtenus démontrent que l'expression des protéines HSP 70 est amplifiée suite au stress UV. En revanche, en présence d'acide rétinoïque, cette expression est nettement diminuée et, de ce fait, les défenses de la peau sont réduites.

L'association avec l'extrait de l'exemple 1 permet de rétablir le taux de protéines HSP, la peau pouvant ainsi se protéger contre le stress UV.

Alors que les rétinoïdes diminuent les défenses de la peau face aux stress extérieurs, l'association avec l'extrait de l'exemple 1 permet de protéger la peau traitée avec les rétinoïdes pendant les stress.

### Exemple 5 - Effets des rétinoïdes et de l'extrait de l'exemple 1 sur l'inflammation induite par les rétinoïdes.

L'extrait d'Artémia de l'exemple 1 a été utilisé à 3 % dans des tests *in vitro* afin de mettre en évidence les bénéfices de son application, en association avec les rétinoïdes, afin de diminuer l'inflammation induite par ceux-ci.

Des kératinocytes humains HaCat et des fibroblastes ont été cultivés dans des boîtes de culture de 100 mm de diamètre. Lorsque les cellules ont atteint 50 % de confluence, des concentrations de 3 % de l'extrait de l'exemple 1 ont été, ou non, appliquées sur les cellules pendant 48 heures. Parallèlement, des concentrations de 10⁻⁷ M d'acide *all-trans* rétinoïque (ATRA) ont été, ou non, appliquées.

Selon les boîtes, différentes conditions ont donc été réalisées :
- Condition contrôle (pas de traitement),
- Extrait de l'exemple 1 pendant 48 heures,
- Acide *all-trans* rétinoïque (ATRA) pendant 6 jours,
- Acide *all-trans* rétinoïque (ATRA) pendant 6 jours dont 48 heures avec l'extrait de l'exemple 1.

Après les 6 jours de culture, le dosage de l'interleukine-1 alpha total a été réalisé par la technique ELISA afin de mettre en évidence les réactions inflammatoires.

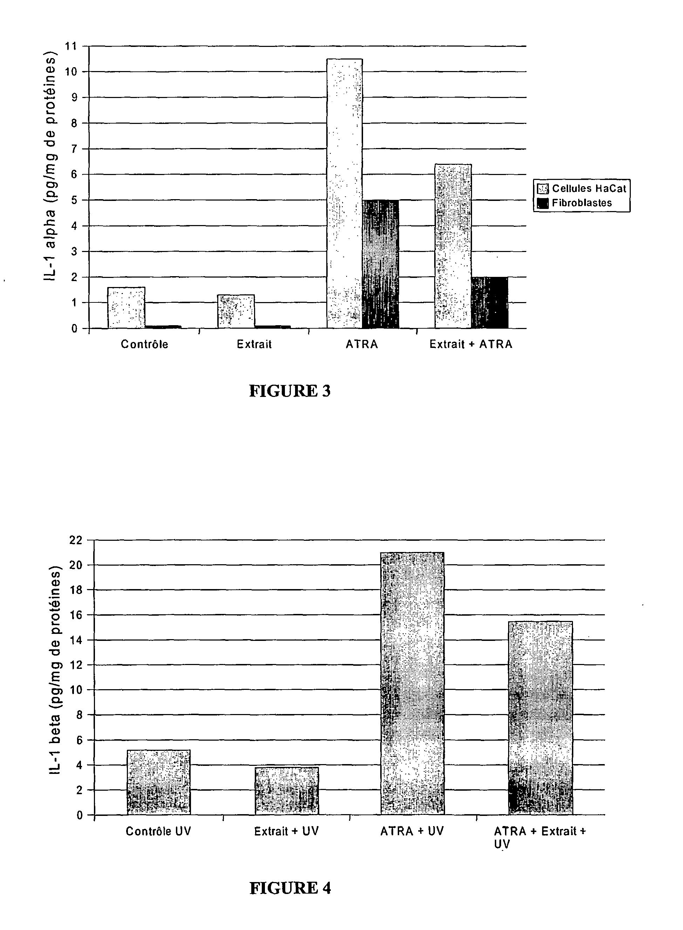

Les résultats obtenus sont illustrés par la figure 3. Ils présentent le dosage de l'interleukine-1 en fonction des différentes conditions de culture. Ils démontrent qu'alors que l'extrait de l'exemple 1 ne modifie pas la réponse inflammatoire, le traitement avec l'acide rétinoïque augmente significativement la concentration en IL-1 alpha. De plus, l'association entre l'acide rétinoïque et l'extrait de l'exemple 1 permet de conserver un taux physiologique d'IL-1 alpha.

### Exemple 6 - Effets des rétinoïdes et de l'extrait de l'exemple 1 sur l'inflammation induite par les UVB.

Des fibroblastes humains en culture ont été soumis à un stress par irradiation. Les fibroblastes ont ensuite été irradiés par un rayonnement UVB à 100 mJ/cm².

Ces fibroblastes irradiés ont été cultivés dans différentes conditions :
- Condition contrôle (Cellules irradiées non traitées),
- Cellules irradiées traitées avec 3 % de l'extrait de l'exemple 1,
- Cellules irradiées traitées avec 10⁻⁷ M d'acide rétinoïque (ATRA),
- Cellules irradiées traitées avec 10⁻⁷ M d'acide rétinoïque et avec 3 % de l'extrait de l'exemple 1.

Après les plusieurs jours de culture, le dosage de l'IL-1 bêta total a été réalisé par la technique ELISA afin de mettre en évidence les réactions inflammatoires. Les résultats obtenus sont illustrés à la figure 4. Ces résultats présentent le dosage de la quantité d'IL-1 bêta en fonction des différentes conditions de culture et après une irradiation avec 100 mJ/cm² d'UVB.

Ces résultats démontrent que le traitement des cellules par l'extrait de l'exemple 1 ne modifie pas la réponse inflammatoire des cellules qui ont été soumises à un rayonnement UVB. Ces résultats démontrent cependant que les cellules, soumises à un stress UV, et qui ont été traitées par des rétinoïdes développent une réaction inflammatoire importante. Ces résultats établissent de plus que, pour les cellules irradiées traitées par des rétinoïdes et par l'extrait de l'exemple 1, le niveau d'IL-1 bêta est plus faible. Il y a donc une diminution de la réponse inflammatoire

Le composé inducteur de protéines HSP permet donc de diminuer l'inflammation induite par les rétinoïdes et par le rayonnement UVB. Il protége ainsi les cellules des stress d'origines extérieures lors les thérapies utilisant les rétinoïdes.

### Exemple 7 - Préparation d'une composition

Cette composition est obtenue par simple mélange des différents composants. Les quantités indiquées sont données en pourcentage de poids.

### Emulsion huile-dans-eau:

### Phase huileuse :

■ Montanov 68 (Cetearyl Alcohol and Cetearyl Glucoside) 5,00 %
■ Huile de Jojoba 5,00 %
■ Acide rétinoïque 0,50 %
■ Huile de Vaseline 5,00 %
■ Isopropyl Palmitate 7,00 %

### Phase aqueuse :

■ Glycérine 5,00 %
■ Allantoïne 0,10 %
■ Extrait de l'exemple 1 5,00 %
■ Sepigel 305 (Polyacrylamide and C13-14 Isoparaffin and Laureth-7) 0,30 %
■ Conservateur 0,50 %
■ Parfum 0,50 %
■ Eau qsp 100 %

## Revendications

1. Composition cosmétique ou pharmaceutique ou dermatologique comprenant, dans un milieu cosmétiquement ou pharmaceutiquement acceptable, au moins un rétinoïde, **caractérisée en ce qu'**elle comprend en outre au moins un inducteur de protéines HSP, ledit inducteur de protéines HSP étant un extrait de zooplancton de l'espèce *Artemia salina,* et **en ce que** le rétinoïde est choisi parmi le groupe comprenant l'acide tout-trans rétinoïque, l'acide 13- cis-rétinoïque, l'acide 9-cis-rétinoïque, les rétinaldéhydes, leurs sels ou leurs esters.

2. Composition selon la revendication 1, **caractérisée en ce qu'**elle se présente sous la forme d'une composition cosmétique et/ou dermatologique adaptée à l'administration par voie topique cutanée.

3. Composition selon l'une des revendications 1 ou 2 **caractérisée en ce que** l'extrait *d'Artemia salina* est obtenu à partir de l'espèce *Artemia salina* en état de dormance.

4. Composition selon l'une des revendications précédentes **caractérisée en ce que** l'extrait *d'Artemia salina* contient au moins 150 mg/litre de diguanosine tétraphosphate.

5. Composition selon l'une quelconque des revendications 1 à 4, **caractérisée en ce que** l'inducteur de protéines HSP est préalablement solubilisé dans un ou plusieurs solvants cosmétiquement ou pharmaceutiquement acceptables comme l'eau, la vaseline, une huile végétale, le propylène glycol, le butylène glycol, le dipropylène glycol, les diglycols éthoxylés ou propoxylés, l'éthanol, le propanol ou l'isopropanol.

6. Composition selon l'une quelconque des revendications 1 à 5, **caractérisée en ce que** l'inducteur de protéines HSP est préalablement solubilisé dans un vecteur cosmétique ou pharmaceutique comme les liposomes ou adsorbé sur des polymères organiques poudreux, des supports minéraux comme les talcs et bentonites, et plus généralement solubilisé dans ou fixé sur, tout vecteur cosmétiquement ou pharmaceutiquement acceptable.

7. Composition selon l'une quelconque des revendications 1 à 6, **caractérisée en ce que** la concentration en composés rétinoïdes est comprise entre 0,0001 % et 5 % du poids total de la composition, et préférentiellement en une quantité représentant de 0,001 et 1 % du poids total de la composition.

8. Composition selon l'une quelconque des revendications 1 à 7 **caractérisée en ce que** l'inducteur de protéines HSP est présent en une quantité représentant de 0,0001 % à 20 % du poids total de la composition, et préférentiellement en une quantité représentant de 0,001 % à 5 % du poids total de la composition.

9. Utilisation d'une quantité efficace d'au moins un composé inducteur de HSP, ledit inducteur de protéines HSP étant un extrait de zooplancton de l'espèce *Artemia salina,* pour la préparation d'une composition, **caractérisée en ce que** le composé ou la composition sont destinés à limiter les effets secondaires des rétinoïdes, et **en ce que** les rétinoïdes sont choisis parmi l'acide tout-trans rétinoïque, l'acide 13-cis- rétinoïque, l'acide 9-cis-rétinoïque, les rétinaldéhydes, leurs sels et/ou leurs esters.

10. Utilisation d'une quantité efficace d'au moins un composé inducteur de HSP, ledit inducteur de protéines HSP étant un extrait de zooplancton de l'espèce *Artemia salina,* pour la préparation d'une composition, **caractérisée en ce que** le composé ou la composition sont destinés à maintenir un taux physiologique de protéines HSP dans les cellules et/ou dans la peau lors d'un traitement par des rétinoïdes, et **en ce que** les rétinoïdes sont choisis parmi le groupe comprenant l'acide tout-trans rétinoïque, l'acide 13-cis- rétinoïque, l'acide 9-cis-rétinoïque, les rétinaldéhydes, leurs sels et/ou leurs esters.

11. Utilisation selon la revendication 10 **caractérisée en ce que** les cellules et/ou la peau sont âgées.

12. Utilisation d'une quantité efficace d'au moins un composé inducteur de HSP, ledit inducteur de protéines HSP étant un extrait de zooplancton de l'espèce *Artemia salina,* pour la préparation d'une composition, **caractérisée en ce que** le composé ou la composition sont destinés à régulariser le taux de cytokines inflammatoires des cellules et/ou de la peau lors d'un traitement par des rétinoïdes, et **en ce que** les rétinoïdes sont choisis parmi le groupe comprenant l'acide tout-trans rétinoïque, l'acide 13-cis- rétinoïque, l'acide 9-cis-rétinoïque, les rétinaldéhydes, leurs sels et/ou leurs esters.

13. Utilisation d'une quantité efficace d'au moins un composé inducteur de HSP, ledit inducteur de protéines HSP étant un extrait de zooplancton de l'espèce *Artemia salina,* pour la préparation d'une composition, **caractérisée en ce que** le composé ou la composition sont destinés à protéger les cellules et/ou la peau des stress d'origines extérieures durant les thérapies utilisant des rétinoïdes, et **en ce que** les rétinoïdes sont choisis parmi le groupe comprenant l'acide tout-trans rétinoïque, l'acide 13-cis- rétinoïque, l'acide 9-cis-rétinoïque, les rétinaldéhydes, leurs sels et/ou leurs esters.

14. Utilisation selon l'une des revendications 10 à 13 **caractérisée en ce que** l'extrait *d'Artemia salina* est obtenu à partir de l'espèce *Artemia salina* en état de dormance.

15. Utilisation selon l'une des revendications 10 à 14 **caractérisée en ce que** l'extrait *d'Artemia salina* contient au moins 150 mg/litre de diguanosine tétraphosphate.

16. Utilisation selon l'une quelconque des revendications 10 à 15 **caractérisée en ce que** le composé inducteur de protéines HSP induit des HSP qui appartiennent à une ou à plusieurs des familles de protéines telles que les petites HSP (10-30 kDa), les HSP40, les HSP60, les HSP70, les HSP90 ou les HSP100-110.

17. Procédé de traitement cosmétique destiné à traiter les signes cutanés du vieillissement et/ou à protéger la peau contre tous les types d'agressions d'origine extérieure et/ou à apporter des soins à la peau, **caractérisé par le fait que** l'on applique sur la peau une composition cosmétique telle que définie selon l'une quelconque des revendications 1 à 8.

## Claims

1. Cosmetic or pharmaceutical or dermatological formulation comprising, in a cosmetically or pharmaceutically acceptable medium, at least one retinoid, **characterised in that** it also comprises at least one HSP protein inducer, said HSP protein inductor being an *Artemia salina* species zooplankton extract, and **in that** the retinoid is selected from the group comprising all-trans retinoic acid, 13-cis-retinoic acid, 9-cis-retinoic acid, retinaldehydes, the salts or esters thereof.

2. Formulation according to claim 1, **characterised in that** it is presented in the form of a cosmetic and/or dermatological formulation suitable for cutaneous topical administration.

3. Formulation according to any of claims 1 or 2 **characterised in that** the *Artemia salina* extract is obtained from the *Artemia salina* species in the dormant state.

4. Formulation according to any of the above claims **characterised in that** the *Artemia salina* extract contains at least 150 mg/litre of diguanosine tetraphosphate.

5. Formulation according to any of claims 1 to 4, **characterised in that** the HSP protein inductor is previously solubilised in one or more cosmetically or pharmaceutically acceptable solvents such as water, vaseline, vegetable oil, propylene glycol, butylene glycol, dipropylene glycol, ethoxylated or propoxylated diglycols, ethanol, propanol or isopropanol.

6. Formulation according to any of claims 1 to 5, **characterised in that** the HSP protein inductor is previously solubilised in a cosmetic or pharmaceutical vector such as liposomes or adsorbed on organic polymer powders, mineral substrates such as talcs and bentonites, and more generally solubilised in or fixed on, any cosmetically or pharmaceutically acceptable vector.

7. Formulation according to any of claims 1 to 6, **characterised in that** the retinoid compound concentration is between 0.001% and 5% of the total weight of the formulation, and preferentially in a quantity representing between 0.001 and 1% of the total weight of the formulation.

8. Formulation according to any of claims 1 to 7 **characterised in that** the HSP protein inductor is present in a quantity representing between 0.0001 % and 20% of the total weight of the formulation and preferentially in a quantity representing between 0.001% and 5% of the total weight of the formulation.

9. Use of an effective quantity of at least one HSP inductor, said HSP inductor being an *Artemia salina* species zooplankton extract, for preparing a formulation, **characterised in that** the compound or formulation is intended to limit the side-effects of retinoids, and **in that** the retinoids are selected from all-trans retinoic acid, 13-cis-retinoic acid, 9-cis-retinoic acid, retinaldehydes, the salts and/or esters thereof.

10. Use of an effective quantity of at least one HSP inductor, said HSP inductor being an *Artemia salina* species zooplankton extract, for preparing a formulation, **characterised in that** the compound or formulation is intended to maintain a physiological HSP protein level in cells and/or skin during retinoid treatment, and **in that** the retinoids are selected from the group comprising all-trans retinoic acid, 13-cis-retinoic acid, 9-cis-retinoic acid, retinaldehydes, the salts and/or esters thereof.

11. Use according to claim 10, **characterised in that** the cells and/or skin are aged.

12. Use of an effective quantity of at least one HSP inductor compound, said HSP inductor being an *Artemia salina* species zooplankton extract, for preparing a formulation, **characterised in that** the compound or formulation is intended to regularise the inflammatory cytokine level of cells and/or skin during retinoid treatment, and **in that** the retinoids are selected from the group comprising all-trans retinoic acid, 13-cis-retinoic acid, 9-cis-retinoic acid, retinaldehydes, the salts and/or esters thereof.

13. Use of an effective quantity of at least one HSP inductor compound, said HSP protein inductor being an *Artemia salina* species zooplankton extract, for preparing a formulation, **characterised in that** the compound or formulation is intended to protect cells and/or skin from stress of external origins during treatments using retinoids, and **in that** the retinoids are selected from the group comprising all-trans retinoic acid, 13-cis-retinoic acid, 9-cis-retinoic acid, retinaldehydes, the salts and/or esters thereof.

14. Use according to any of claims 10 to 13 **characterised in that** the *Artemia salina* extract is obtained from the *Artemia salina* species in the dormant state.

15. Use according to any of claims 10 to 14 **characterised in that** the *Artemia salina* extract contains at least 150 mg/litre of diguanosine tetraphosphate.

16. Use according to any of claims 10 to 15 **characterised in that** the HSP protein inductor compound induces HSPs belonging to one or more protein families such as small HSPs (10-30 kDa), HSP40, HSP60, HSP70, HSP90 or HSP100-110.

17. Cosmetic treatment method intended to treat signs of skin ageing and/or protect skin against any types of attacks of external origin and/or provide skin treatments, **characterised in that** a cosmetic formulation as defined according to any of claims 1 to 8 is applied to the skin.

## Patentansprüche

1. Kosmetische oder pharmazeutische oder dermatologische Zusammensetzung, in einem kosmetisch oder pharmazeutisch annehmbaren Milieu, mindestens ein Retinoid umfassend, **dadurch gekennzeichnet, dass** sie außerdem mindestens einen Induktor von HSP-Proteinen umfasst, wobei besagter Induktor von HSP-Proteinen ein Zooplanktonextrakt der Spezies *Artemia salina* ist, und dass, das Retinoid ausgewählt ist aus der Gruppe, die die all-trans-Retinsäure, die 13-cis-Retinsäure, die 9-cis-Retinsäure, die Retinaldehyde, deren Salze und deren Ester umfasst.

2. Zusammensetzung nach Anspruch 1, **dadurch gekennzeichnet, dass** sie sich in der Form einer kosmetischen und/oder dermatologischen Zusammensetzung darstellt, die zur Verabreichung auf topischem Weg über die Haut geeignet ist.

3. Zusammensetzung nach einem der Ansprüche 1 oder 2, **dadurch gekennzeichnet, dass** das Extrakt von *Artemia salina* aus der Spezies *Artemia salina* im Ruhezustand gewonnen wird.

4. Zusammensetzung nach einem der vorhergehenden Ansprüche, **dadurch gekennzeichnet, dass** das Extrakt von *Artemia salina* mindestens 150 mg/Liter Diguanosintetraphosphat enthält .

5. Zusammensetzung nach einem der Ansprüche 1 bis 4, **dadurch gekennzeichnet, dass** der Induktor von HSP-Proteinen zuvor in einem oder mehreren kosmetisch oder pharmazeutisch akzeptablen Lösungsmitteln wie Wasser, Vaseline, einem Pflanzenöl, Propylenglykol, Butylenglykol, Dipropylenglykol, den ethoxylierten oder propoxylierten Diglykolen, Ethanol, Propanol oder Isopropanol solubilisiert wird.

6. Zusammensetzung nach einem der Ansprüche 1 bis 5, **dadurch gekennzeichnet, dass** der Induktor von HSP-Proteinen zuvor in einem kosmetischen oder pharmazeutischen Vektor wie den Liposomen solubilisiert oder auf pulverförmigen organischen Polymeren, mineralischen Trägem wie den Talken und Bentonit adsorbiert wird, und allgemeiner in jedem kosmetisch oder pharmazeutisch akzeptablen Vektor solubilisiert oder auf demselben fixiert wird.

7. Zusammensetzung nach einem der Ansprüche 1 bis 6, **dadurch gekennzeichnet, dass** die Konzentration an Retinoidverbindungen zwischen 0,0001% und 5% des Gesamtgewichts der Zusammensetzung ist, und vorzugsweise in einer Menge, die von 0,001 bis 1% des Gesamtgewichts der Zusammensetzung darstellt.

8. Zusammensetzung nach einem der Ansprüche 1 bis 7, **dadurch gekennzeichnet, dass** der Induktor von HSP-Proteinen in einer Menge vorhanden ist, die von 0,0001% bis 20% des Gesamtgewichts der Zusammensetzung darstellt, und vorzugsweise in einer Menge, die von 0,001 % bis 5% des Gesamtgewichts der Zusammensetzung darstellt.

9. Verwendung einer wirksamen Menge mindestens einer HSP induzierenden Verbindung, wobei besagter Induktor von HSP-Proteinen ein Zooplanktonextrakt der Spezies *Artemia salina* ist, für die Zubereitung einer Zusammensetzung, **dadurch gekennzeichnet, dass** die Verbindung oder die Zusammensetzung dazu bestimmt sind, die Nebenwirkungen der Retinoide zu begrenzen und dass, die Retinoide ausgewählt sind aus der all-trans-Retinsäure, der 13-cis-Retinsäure, der 9-cis-Retinsäure, den Retinaldehyden, deren Salzen und deren Estern.

10. Verwendung einer wirksamen Menge mindestens einer HSP induzierenden Verbindung, wobei besagter Induktor von HSP-Proteinen ein Zooplanktonextrakt der Spezies *Artemia salina* ist, für die Zubereitung einer Zusammensetzung, **dadurch gekennzeichnet, dass** die Verbindung oder die Zusammensetzung dazu bestimmt sind, bei einer Behandlung mit Retinoiden einen physiologischen Spiegel von HSP-Proteinen in den Zellen und/oder in der Haut aufrecht zu erhalten, und dass, die Retinoide ausgewählt sind aus der Gruppe, die die alltransRetinsäure, die 13-cis-Retinsäure, die 9-cis-Retinsäure, die Retinaldehyde, deren Salze und/oder deren Ester umfasst.

11. Verwendung nach Anspruch 10, **dadurch gekennzeichnet, dass** die Zellen und/oder die Haut alt sind.

12. Verwendung einer wirksamen Menge mindestens einer HSP induzierenden Verbindung, wobei besagter Induktor von HSP-Proteinen ein Zooplanktonextrakt der Spezies *Artemia salina* ist, für die Zubereitung einer Zusammensetzung, **dadurch gekennzeichnet, dass** die Verbindung oder die Zusammensetzung dazu bestimmt sind, bei einer Behandlung mit Retinoiden den Spiegel entzündlicher Zytokine der Zellen und/oder der Haut zu regulieren, und dass, die Retinoide ausgewählt sind aus der Gruppe, die die all-trans-Retinsäure, die 13-cis-Retinsäure, die 9-cis-Retinsäure, die Retinaldehyde, deren Salze und/oder deren Ester umfasst.

13. Verwendung einer wirksamen Menge mindestens einer HSP induzierenden Verbindung, wobei besagter Induktor von HSP-Proteinen ein Zooplanktonextrakt der Spezies *Artemia salina* ist, für die Zubereitung einer Zusammensetzung, **dadurch gekennzeichnet, dass** die Verbindung oder die Zusammensetzung dazu bestimmt sind, die Zellen und/oder die Haut bei Therapien, die Retinoide verwenden, vor Stress von außen zu schützen, und, dass die Retinoide ausgewählt sind aus der Gruppe, die die all-trans-Retinsäure, die 13-cis-Retinsäure, die 9-cis-Retinsäure, die Retinaldehyde, deren Salze und/oder deren Ester umfasst.

14. Verwendung nach einem der Ansprüche 10 bis 13, **dadurch gekennzeichnet, dass** das Extrakt von *Artemia salina* aus der Spezies *Artemia salina* im Ruhezustand gewonnen wird.

15. Verwendung nach einem der Ansprüche 10 bis 14, **dadurch gekennzeichnet, dass** das Extrakt von *Artemia salina* mindestens 150 mg/Liter Diguanosintetraphosphat enthält.

16. Verwendung nach einem der Ansprüche 10 bis 15, **dadurch gekennzeichnet, dass** die HSP-Proteine induzierende Verbindung HSP induziert, welche zu einer oder mehreren Proteinfamilien gehören wie die kleinen HSP (10-30 kDa), die HSP40, die HSP60, die HSP70, die HSP90 oder die HSP100-110.

17. Verfahren zur kosmetischen Behandlung, das dazu bestimmt ist, die Zeichen der Hautalterung zu behandeln und/oder die Haut vor allen Arten von Aggressionen von außen zu schützen und/oder die Haut zu pflegen, **dadurch gekennzeichnet, dass** man auf die Haut eine kosmetische Zusammensetzung aufträgt, wie nach einem der Ansprüche 1 bis 8 definiert.
